# EUROPÄISCHE PATENTSCHRIFT

(11) **EP 0 995 394 B1**
(45) Veröffentlichungstag und Bekanntmachung des Hinweises auf die Patenterteilung: **10.03.2004**
(21) Anmeldenummer: 99115464.2
(22) Anmeldetag: 05.08.1999
(51) Int. Cl.: A61B 3/18, A61B 3/028

(54) **Untersuchungseinheit für Augenuntersuchungen**
Examination unit for eye examination
Unité d'examen pour l'examen des yeux

(30) Priorität: 24.10.1998 DE 29818992 U
(43) Veröffentlichungstag der Anmeldung: 26.04.2000
(73) Patentinhaber: Oculus Optikgeräte GmbH, 35582 Wetzlar-Dutenhofen (DE)
(72) Erfinder: Feiertag, Carsten, 35410 Hungen (DE)
(74) Vertreter: Böck, Bernhard, Dipl.-Ing.

(56) Entgegenhaltungen:
- DE-A- 3 703 677
- DE-A- 4 237 077
- US-A- 5 444 504
- US-A- 5 777 344

## Beschreibung

Die Erfindung betrifft eine Untersuchungseinheit für Augenuntersuchungen mit einem Patientenstuhl, mit einem mit dem Patientenstuhl verbundenen Ständer, an dem ein höhenverstellbarer und in der wesentlichen Ebene des Arms seitlich verschiebbarer oder an einem Ständer drehbarer Phoropterarm gelagert ist.

Derartige Untersuchungseinheiten sind marktüblich und werden in fast allen augenärztlichen Praxen verwendet. Der Patientenstuhl ist dabei in der Regel über einen Unterbau mit einem Ständer verbunden. An diesem Ständer ist ein verfahrbarer Phoropterarm gelagert. Der an diesem Phoropterarm befestigte Phoropter dient der Bestimmung der Fehlsichtigkeit der Augen des Patienten, der auf dem Patientenstuhl Platz nimmt. Dazu wird der Phoropter vor den Patienten gefahren. Je nach Platzangebot in dem Untersuchungszimmer sind dabei zwei verschiedene Verfahrbewegungen üblich. Platzaufwendiger ist eine Verfahrbewegung des Phoropters quer zum Patienten. Eine andere Möglichkeit ist, dass der Phoropter um 90° von dem Patienten in einer Drehbewegung weggeschwenkt wird. Dabei muss beachtet werden, dass sich in dem Endpunkt der Phoropterbewegung oftmals andere optische Geräte oder ein Monitor auf einem zusätzlichen Gerätetisch, der ebenfalls mit dem Ständer verbunden ist, befinden können. Deshalb muss der Phoropter auch in der Höhe verstellt werden, um einen Endpunkt der Schwenkbewegung oberhalb dieser sich auf dem Gerätetisch befindlichen Geräte zu finden. Neben dieser Verschiebebewegung muss der Phoropter aber auch an die Größe des Patienten angepasst eingestellt werden. Auch deshalb ist der Phoropter bzw. der Phoropterarm höhenverstellbar. Um die beiden Bewegungen zu ermöglichen, sind nach dem Stand der Technik jeweils eigene Lager für jede Bewegung vorgesehen.

Dokument US-A-5 777 344 offenbart eine Vorrichtung mit einem Photopterarm der höhenverstellbar und seitlich verschiebbar ist.

Bei jedem zu untersuchenden Patienten muss eine solche Verfahrbewegung zweimal durchgeführt werden. Zum Ersten, um den Phoropter vor den Patient vorzufahren, zum Zweiten, um den Phoropter wieder in seine Ausgangsposition zurückzuverfahren. Der Untersuchende muss also bei mehreren Patienten pro Tag immer wieder die Verfahrbewegungen ausführen. Dieses führt gerade bei weniger kräftigen Personen zur schnellen Ermüdung und im schlimmsten Fall zu Schäden am Bewegungsapparat.

Die Erfindung hat sich die Aufgabe gestellt, eine Untersuchungseinheit kostengünstig und mit geringem technischem Aufwand so weiterzubilden, dass die Untersuchungseinheit wartungsarm ist und der Phoropterarm so einfach zu verfahren ist, dass der Untersuchende weniger stark ermüdet und Schäden am Bewegungsapparat vorgebeugt ist.

Die Aufgabe wird erfindungsgemäß mit den Merkmalen des Anspruchs 1 gelöst.

Eine bekannte Untersuchungseinheit für Augenuntersuchungen wurde so weiter gebildet, dass nunmehr zwischen dem Ständer und dem Phoropterarm ein einziges Lager vorhanden ist und so der Phoropterarm mittels einer einzigen Bewegung von der Position vor dem Patienten in die zweite Endposition gebracht werden kann. Das Lager ist eine Führung, die die Bewegung nur entlang einer definierten Bahnkurve erlaubt. Diese Bahnkurve weist zumindest eine translatorische und eine rotatorische Komponente oder aber zwei translatorische Komponenten auf. Im Gegensatz zum Stand der Technik kann nun auch der Phoropterarm durch einen einzigen Antrieb verfahren werden.

Vorteilhaft ist der Phoropterarm parallel zu einer Hochachse des Ständers höhenverschiebbar und seitlich parallel zu seiner wesentlichen Ebene vor den Patientenstuhl hinschiebbar in einer Führung gelagert.

In einer Ausführungsform ist der Phoropterarm parallel zu einer Hochachse des Ständers höhenverschiebbar und um die Hochachse schwenkbar in einer Führung gelagert. Gemäß der Erfindung weist der Phoropterarm eine Buchse auf. Diese Buchse wird von der Hochachse des Ständers durchgriffen und zwischen Ständer und Buchse ist die Führung ausgebildet, wobei eine Nase an dem Ständer in einen Schlitz der Buchse eingreift. Vorteilhaft ist dann die Führung zumindest abschnittsweise in Wendelform um diese Säule gelegt.

Erfindungsgemäß kann der Antrieb entweder ortsfest zum Ständer oder aber zum Phoropterarm angebracht sein. Der Antrieb kann ein Motor sein, welcher mit einem Seilzug den Phoropterarm verfährt. Dieser Seilzug läuft vorteilhaft über eine oder mehrere Umlenkrollen, wobei die Umlenkrolle oberhalb der Buchse an dem Ständer angebracht ist und der Seilzug einen spitzen Winkel zur Hochachse des Ständers bildet. In einer anderen Ausführungsform kann der Antrieb auch ein linear wirkender Antrieb sein, beispielsweise eine Druckzylindereinheit.

Gemäß der Erfindung kann die Untersuchungseinheit einen Gerätetisch aufweisen, welcher mit dem Ständer verbunden ist und weitere augenärztliche Untersuchungsgeräte, beispielsweise eine Spaltlampe und/oder ein Ophthalmometer und/oder ein Keratometer trägt. Dieser Gerätetisch ist von Hand oder durch einen zweiten Antrieb vor dem Patientenstuhl verfahrbar. Hierbei ist die Verfahrbewegung eine Schwenkbewegung oder eine Verschiebebewegung.

Ein Ausführungsbeispiel ist anhand der Zeichnung näher beschrieben. Darin zeigt
- Fig. 1: eine Ansicht einer Untersuchungseinheit
und
- Fig. 2: eine Detailansicht einer Führung der Untersuchungseinheit gemäß Figur 1.

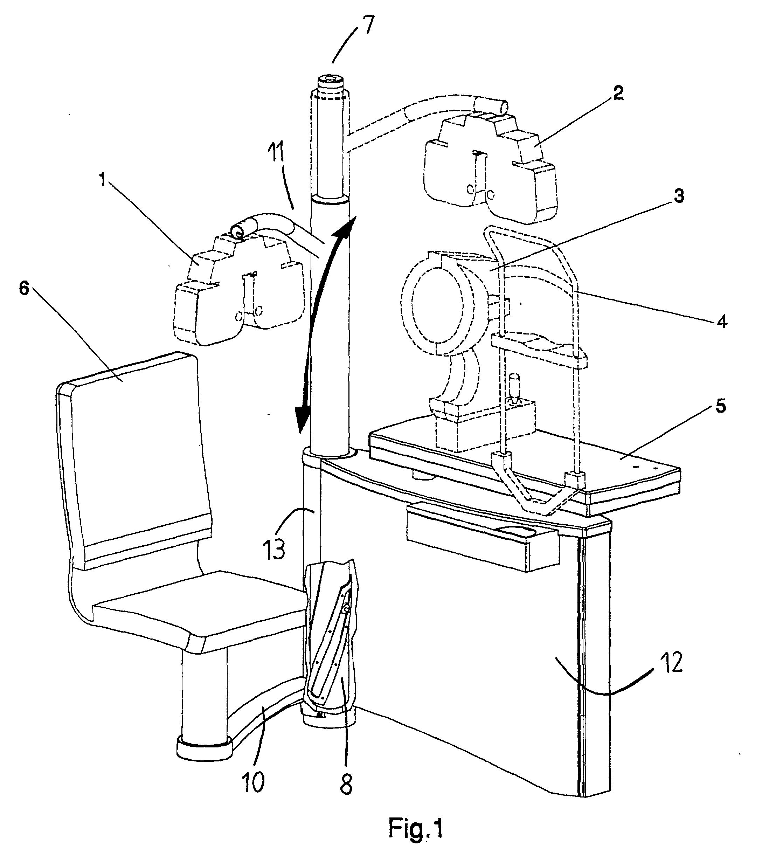

Bei der in Figur 1 dargestellten Untersuchungseinheit ist der Patientenstuhl 6 über einen Unterbau 10 mit einer Säule 7 verbunden. An dieser Säule 7 ist ein Phoropterarm 11 verfahrbar gelagert. An diesem Phoropterarm 11 ist der Phoropter 1, 2 befestigt. Dargestellt ist ein Phoropter in zwei verschiedenen Stellungen. In Stellung 1 befindet sich der Phoropter in der Untersuchungsposition vor dem Patienten (Untersuchungsstellung), in Stellung 2 befindet sich der Phoropter in der zweiten Endstellung (Normalstellung).

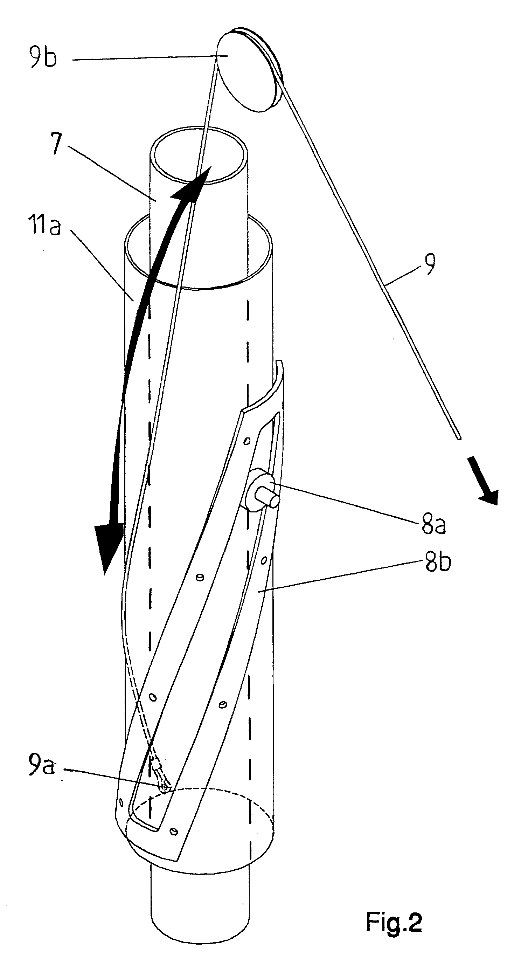

Der untere Teil der Säule 7 ist durch eine Blende 13 verdeckt. In einem Ausbruch in der Blende 13 lässt sich die Führung 8 des Phoropterarms an der Säule 7 erkennen. Eine Detailansicht der Führung 8 ist Figur 2 zu entnehmen. Wie aus Figur 1 weiter zu erkennen, ist ein Gerätetisch 5 über einen zweiten Unterbau 12 mit der Säule verbunden. Auf dem Gerätetisch 5 können weitere Geräte 3,4 angebracht werden. Ebenfalls kann in dem zweiten Unterbau 12 eine Rechnereinheit integriert sein.

Die Führung 8 (Figur 2) ist folgendermaßen konstruiert. Die Säule 7 ist in eine Buchse 11a des Phoropterarms 11 mit Spiel eingesetzt. Die Säule 7 ist ortsfest, während das Rohr 11a gegenüber der Standfläche der Untersuchungseinheit verschiebbar ist. Die Führung 8 wird durch einen wendelförmigen Schlitz 8b in der Buchse 11a gebildet, in das eine Nase 8a, die auf der Säule 7 befestigt ist, eingreift.

Die Verfahrbewegung des Phoropterarms 11 erfolgt durch einen nicht dargestellten, ortsfesten Motor und einen Seilzug 9 zwischen Motor und Buchse 11a. Der Seilzug 9 ist im Punkt 9a an der Buchse 11a befestigt, wird über die Umlenkrolle 9b umgelenkt und vom Motor gezogen. Dabei ist die Umlenkrolle 9b oberhalb der Buchse 11a am Ständer 7 angebracht und der Seilzug verläuft in einem spitzen Winkel zur Hochachse des Ständers 7. Zieht nun dieser Motor an dem Seil und der Buchse 11a und befinden diese sich zuvor in der in Figur 2 dargestellten Position, so bewegt sich die Buchse 11a nach oben und führt gleichzeitig eine Bewegung entgegen dem Uhrzeigersinn aus. Der an dem Phoropterarm 11 befestigte Phoropter bewegt sich dann aus der Position 1 in die Position 2 (Figur 1). Der Motor selbst befindet sich vorteilhafterweise in dem Unterbau 12.

### Bezugszeichenliste:

- 1 -: Phoropter in der Untersuchungsstellung
- 2 -: Phoropter in der Normalstellung
- 3,4 -: weitere optische Geräte
- 5 -: Gerätetisch
- 6 -: Patientenstuhl
- 7 -: Säule
- 8 -: Führung
- 8a -: Nase
- 8b -: Schlitz
- 9 -: Seilzug
- 9a -: Anschlag des Seilzugs
- 9b -: Umlenkrolle
- 10 -: erster Unterbau
- 11 -: Phoropterarm
- 11a -: Buchse
- 12 -: zweiter Unterbau
- 13 -: Blende

## Patentansprüche

1. Untersuchungseinheit für Augenuntersuchungen mit einem Patientenstuhl, mit einem mit dem Patientenstuhl verbundenen Ständer, an dem ein höhenverstellbarer und in der wesentlichen Ebene des Arms (11) seitlich verschiebbarer oder an einem Ständer (7) drehbarer Phoropterarm gelagert ist,
**dadurch gekennzeichnet,**
**dass** der Phoropterarm (11) in einer Führung (8) derart gelagert ist, dass mittels eines einzigen Antriebes bei einer Verschwenkung des Phoropterarmes dieser in der Höhe verstellt wird.

2. Untersuchungseinheit nach Anspruch 1, **dadurch gekennzeichnet, dass** der Phoropterarm (11) parallel zu einer Hochachse des Ständers 7 höhenverschiebbar und seitlich parallel zu seiner wesentlichen Ebene vor den Patientenstuhl verschiebbar in einer Führung (8) gelagert ist.

3. Untersuchungseinheit nach Anspruch 1, **dadurch gekennzeichnet, dass** der Phoropterarm (11) parallel zu einer Hochachse des Ständers (7) höhenverschiebbar und um die Hochachse schwenkbar in einer Führung (8) gelagert ist.

4. Untersuchungseinheit nach Anspruch 3, **dadurch gekennzeichnet, dass** der Phoropterarm (11) eine Buchse (11a) aufweist, dass die Hochachse des Ständers (7) die Buchse (11a) durchgreift und dass die Führung (8) zwischen Buchse (11a) und Ständer (8) durch eine Nase (8a) des Ständers (7) gebildet wird, die in einen Schlitz (8b) der Buchse (11a) eingreift.

5. Untersuchungseinheit nach Anspruch 4, **dadurch gekennzeichnet, dass** die Führung (8) zumindest abschnittsweise wendelförmig um die Säule (7) gelegt ist.

6. Untersuchungseinheit nach Anspruch 1 bis 5, **dadurch gekennzeichnet, dass** der Antrieb ortsfest zum Ständer (7) oder zum Phoropterarm (11) angebracht ist.

7. Untersuchungseinheit nach Anspruch 1 bis 6, **dadurch gekennzeichnet, dass** der Antrieb einen Motor und einen Seilzug (9) aufweist.

8. Untersuchungseinheit nach Anspruch 7, **dadurch gekennzeichnet, dass** das Seil des Seilzuges (9) über mindestens eine Umlenkrolle (9b) läuft, wobei die Umlenkrolle oberhalb der Buchse (11a) am Ständer (7) angebracht ist und das Seil in einem spitzen Winkel zur Hochachse des Ständers (7) verläuft.

9. Untersuchungseinheit nach Anspruch 1 bis 6, **dadurch gekennzeichnet, dass** der Antrieb ein linear wirkender Antrieb ist.

10. Untersuchungseinheit nach Anspruch 9, **dadurch gekennzeichnet, dass** der Antrieb ein Druckzylinder ist.

11. Untersuchungseinheit nach einem der Ansprüche 1 bis 10, **dadurch gekennzeichnet, dass** ein Gerätetisch (5) mit dem Ständer verbunden ist und weitere augenärztliche Untersuchungsgeräte (3,4) beispielsweise eine Spaltlampe und/oder ein Ophthalmometer und/oder ein Keratometer auf dem Gerätetisch (5) angebracht sind.

12. Untersuchungseinheit nach Anspruch 11, **dadurch gekennzeichnet, dass** der Gerätetisch (5) vor dem Patientenstuhl (6) verschwenkbar oder verschiebbar ist.

13. Untersuchungseinheit nach einem der Ansprüche 1 bis 12, **dadurch gekennzeichnet, dass** in die Untersuchungseinheit eine Rechnereinheit integriert ist.

## Claims

1. An examination unit for eye examinations comprising a patient's chair, comprising a stand connected to the patient's chair, on which there is mounted a phoropter arm which is vertically adjustable and laterally displaceable in the fundamental plane of the arm (11) or is rotatable on a stand (7),
**characterised**
**in that** the phoropter arm (11) is mounted in a guide (8) such that as the phoropter arm pivots, it is adjusted in height by means of a single drive mechanism.

2. An examination unit according to Claim 1, **characterised in that** the phoropter arm (11) is mounted in a guide (8) so as to be vertically adjustable parallel to a vertical axis of the stand (7) and displaceable laterally parallel to its fundamental plane in front of the patient's chair.

3. An examination unit according to Claim 1, **characterised in that** the phoropter arm (11) is mounted in a guide (8) so as to be vertically adjustable parallel to a vertical axis of the stand (7) and pivotable about the vertical axis.

4. An examination unit according to Claim 3, **characterised in that** the phoropter arm (11) has a bushing (11a), **in that** the vertical axis of the stand (7) extends through the bushing (11a), and **in that** the guide (8) between the bushing (11a) and the stand (8) [sic, recte (7)] is formed by a lug (8a) of the stand (7) which engages into a slot (8b) of the bushing (11a).

5. An examination unit according to Claim 4, **characterised in that** at least a portion of the guide (8) is placed in a helical shape around the column (7).

6. An examination unit according to Claim 1 to 5, **characterised in that** the drive mechanism is mounted so as to be fixed in relation to the stand (7) or to the phoropter arm (11).

7. An examination unit according to Claim 1 to 6, **characterised in that** the drive mechanism has a motor and a cable line (9).

8. An examination unit according to Claim 7, **characterised in that** the cable of the cable line (9) runs via at least one deflection pulley (9b), the deflection pulley being mounted above the bushing (11a) on the stand (7) and the cable extending at an acute angle to the vertical axis of the stand (7).

9. An examination unit according to Claim 1 to 6, **characterised in that** the drive mechanism is a linearly operating drive mechanism.

10. An examination unit according to Claim 9, **characterised in that** the drive mechanism is a pressure cylinder.

11. An examination unit according to one of Claims 1 to 10, **characterised in that** an instrument table (5) is connected to the stand and other ophthalmic examination instruments (3, 4), for instance a slit lamp and/or an ophthalmometer and/or a keratometer, are mounted on the instrument table (5).

12. An examination unit according to Claim 11, **characterised in that** the instrument table (5) is pivotable or displaceable in front of the patient's chair (6).

13. An examination unit according to one of Claims 1 to 12, **characterised in that** a computer unit is incorporated in the examination unit.

## Revendications

1. Unité d'examen pour l'examen des yeux avec un siège de patient, avec une colonne reliée au siège du patient, sur laquelle est logé un bras de phoromètre réglable en hauteur, de manière à pouvoir coulisser latéralement dans le plan principal du bras (11), ou de manière à pouvoir pivoter sur une colonne (7),
**caractérisée en ce que**,
le bras de phoromètre (11) est logé dans un guide (8), de telle sorte que celui-ci est réglé en hauteur au moyen d'un seul entraînement lors d'un déplacement horizontal du bras de phoromètre.

2. Unité d'examen selon la revendication 1, **caractérisée en ce que** le bras de phoromètre (11) est logé dans un guide (8), de manière à pouvoir coulisser en hauteur parallèlement à un axe vertical de la colonne 7 et à pouvoir coulisser à l'avant du siège du patient latéralement et parallèlement à son plan principal.

3. Unité d'examen selon la revendication 1, **caractérisée en ce que** le bras de phoromètre (11) est logé dans un guide (8) de manière à pouvoir coulisser en hauteur parallèlement à un axe vertical de la colonne (7) et de manière à pouvoir pivoter autour de l'axe vertical.

4. Unité d'examen selon la revendication 3, **caractérisée en ce que** le bras de phoromètre (11) comprend un manchon (11a), que l'axe vertical de la colonne (7) traverse le manchon (11a), et que le guide (8) entre le manchon (11a) et la colonne (8) est formé par un nez (8a) de la colonne (7) qui s'engage dans une fente (8b) du manchon (11a).

5. Unité d'examen selon la revendication 4, **caractérisée en ce que** le guide (8) est disposé au moins par partie hélicoïdalement autour de la colonne (7).

6. Unité d'examen selon la revendication 1 à 5, **caractérisée en ce que** l'entraînement est monté de façon fixe par rapport à la colonne (7) ou au bras de phoromètre (11).

7. Unité d'examen selon la revendication 1 à 6, **caractérisée en ce que** l'entraînement comprend un moteur et une commande par câble (9).

8. Unité d'examen selon la revendication 7, **caractérisée en ce que** le câble de la commande par câble (9) passe par au moins une poulie de renvoi (9b), la poulie de renvoi étant placée au dessus du manchon (11a) sur la colonne (7) et le câble s'étendant dans un angle aigu par rapport à l'axe vertical de la colonne (7).

9. Unité d'examen selon la revendication 1 à 6, **caractérisée en ce que** l'entraînement est un entraînement à action linéaire.

10. Unité d'examen selon la revendication 9, **caractérisée en ce que** l'entraînement est un vérin pneumatique.

11. Unité d'examen selon l'une des revendications 1 à 10, **caractérisée en ce qu'**une table d'appareillage (5) est reliée à la colonne, et d'autres appareils d'examen oculaire (3,4), par exemple une lampe à fente et/ou un ophtalmomètre et/ou un kératomètre, sont fixés sur la table d'appareillage (5).

12. Unité d'examen selon la revendication 11, **caractérisée en ce que** la table d'appareillage (5) peut être coulissée ou pivotée devant le siège du patient (6).

13. Unité d'examen selon l'une des revendications 1 à 12, **caractérisée en ce qu'**une unité d'ordinateur est intégrée dans l'unité d'examen.
